# EUROPEAN PATENT APPLICATION

(11) **EP 4 830 721 A1**
(43) Date of publication of application: **28.10.2026**
(21) Application number: 26164211.0
(22) Date of filing: 12.03.2026
(51) Int. Cl.: A61K 40/11, A61K 40/31, A61K 40/42

(54) **CHIMERIC ANTIGEN RECEPTORS AND USE THEREOF**

(30) Priority: 24.04.2025 JP 2025072166
(71) Applicant: Hitachi, Ltd., Tokyo 100-8280 (JP)
(72) Inventor: OKUMA, Atsushi, Tokyo 100-8280 (JP); HISADA, Shoji, Tokyo 100-8280 (JP); YOSHIDA, Kei, Tokyo 100-8280 (JP); ISHIDA, Yoshihito, Tokyo 100-8280 (JP); MIURA, Takuya, Tokyo 100-8280 (JP); KAWARA, Taketo, Tokyo 100-8280 (JP); ITO, Daisuke, Tokyo 100-8280 (JP)
(74) Representative: Mewburn Ellis LLP

(57) **Abstract**

This invention relates to a chimeric antigen receptor (CAR), a nucleic acid sequence encoding the same, a vector and a cell comprising the same, and a pharmaceutical composition. Specifically, this invention relates to a chimeric antigen receptor (CAR) comprising: a single chain variable fragment (scFv) capable of binding to CD19, a hinge, a transmembrane region, and an intracellular region, wherein the hinge comprises the amino acid sequence shown in SEQ ID NO: 5 or 6, and the intracellular region comprises a costimulatory domain comprising the amino acid sequence shown in SEQ ID NO: 8 or 9.

## Description

### BACKGROUND OF THE INVENTION

### Field of the Invention

The present invention relates to a chimeric antigen receptor (CAR), a nucleic acid sequence encoding the same, a vector and a cell comprising the same, and a pharmaceutical composition.

### Background Art

In recent years, approvals of therapeutic methods using T cells with function enhanced through genetic modification, such as chimeric antigen receptor (CAR) T cells (CAR-T), have been increasing. Research and development of CARs, particularly for use in the cancer treatment, have been accelerating.

For example, as a CAR that has shown efficacy in hematologic malignancies, CD19-targeted CAR-T cell therapy products such as Kymriah (R) have been approved (WO 2012/079000 A1). Moreover, for CAR design, use of the 4-1BB intracellular domain (US 8399645 B), and a CAR containing an amino-acid mutation that targets HER2 (WO 2017/079694 A1) have been reported.

### SUMMARY OF THE INVENTION

Among CARs that have shown efficacy in hematologic malignancies, some exhibit strong short-term activity but poor *in vivo* persistence, while others show modest short-term activity but high *in vivo* persistence, and these two parameters are considered to be in a trade-off relationship, and a CAR design that achieves high levels of both the parameters has not been established (for example, Zhao, Z. et al., Cancer Cell 28:415-428, 2015). Thus, there is still need for improvement in medicinal efficacy through optimization of CAR design.

Therefore, an object of the present invention is to provide a CAR having cytotoxic activity and activity persistence.

In order to solve the above-described problem, the present inventors have made earnest studies on design of the hinge and the intracellular domain in CARs targeting CD19. As a result, a CAR improved in cytotoxic activity and activity persistence has been developed by specified mutations in the hinge and the intracellular domain in a CAR.

In a first aspect, the present invention provides a chimeric antigen receptor (CAR) comprising: a single chain variable fragment (scFv) capable of binding to CD19, a hinge, a transmembrane region, and an intracellular region, wherein the hinge comprises the amino acid sequence shown in SEQ ID NO: 5 or 6, and the intracellular region comprises a costimulatory domain comprising the amino acid sequence shown in SEQ ID NO: 8 or 9.

In a second aspect, the present invention provides a nucleic acid sequence encoding the CAR of the present invention. The present invention also provides a vector comprising the nucleic acid sequence of the present invention.

In a third aspect, the present invention provides a T cell that expresses the CAR of the present invention, or comprises the nucleic acid sequence of the present invention.

In a fourth aspect, the present invention provides a composition comprising the CAR of the present invention, the nucleic acid sequence of the present invention, or the vector of the present invention, and a pharmaceutical composition comprising the T cell of the present invention.

The present invention provides a CAR capable of binding to CD19, and having cytotoxic activity and activity persistence. By using this CAR, more effective CAR-T cell therapy can be provided.

### BRIEF DESCRIPTION OF THE DRAWINGS

FIG. 1 is a schematic diagram of an exemplary CAR variant, and a diagram illustrating sequence alignment of the hinge, the transmembrane region, and the costimulatory domain.
FIG. 2 is a graph illustrating cytotoxic activity obtained in the first co-culture of CAR-expressing T cells (Cycle 1) and subsequent co-cultures using the serially diluted cultures (Cycles 2 to 5) in Example 2. Error bars indicate standard deviation.
FIG. 3 is a graph illustrating expression, in CAR-expressing T cells, of cell surface markers Naive/SCM (Naive), CM, EM, and TERMA obtained in Example 3. Error bars indicate standard deviation.
FIG. 4 is a graph illustrating the tumor shrinkage effect and a survival rate achieved by CAR-expressing T cells obtained in Example 4. The arrow indicates time point of intravenous injection of CAR-expressing T cells or PBS, and error bars indicate standard deviation.
FIG. 5 shows a vector map of an exemplary CAR-expressing plasmid vector.

### DETAILED DESCRIPTION OF THE PREFERRED EMBODIMENTS

The present invention relates to a chimeric antigen receptor (CAR) targeting CD19, a nucleic acid sequence encoding the same, a vector and a cell comprising the same, and a pharmaceutical composition. Specifically, the present invention is based on the finding that when a CD8α-derived hinge having specific mutations and a 4-1BB-derived intracellular domain (costimulatory domain) having specific mutations are used in a CAR, short-term cytotoxic activity is improved and equivalent *in vivo* persistence is exhibited as compared to a CAR using the original hinge and intracellular domain (costimulatory domain). A CAR having such mutations is herein referred to also as the "CAR variant".

### 1. Chimeric Antigen Receptor (CAR)

A first aspect of the present invention is a chimeric antigen receptor (CAR). Specifically, the present invention provides a chimeric antigen receptor (CAR), comprising a single chain variable fragment (scFv) capable of binding to CD19, a hinge, a transmembrane region, and an intracellular region, wherein the hinge comprises the amino acid sequence shown in SEQ ID NO: 5 or 6, and the intracellular region comprises a costimulatory domain comprising the amino acid sequence shown in SEQ ID NO: 8 or 9. A T cell into which the CAR of the present invention has been introduced has cytotoxic activity against a cell having CD19 on the surface, such as a cancer cell.

The following terms frequently used herein are defined as follows.

"Chimeric antigen receptor T-cell therapy (CAR-T cell therapy)" refers to a treatment method that includes introducing a chimeric antigen receptor (CAR) gene into T cells or the like collected from the peripheral blood of a donor to prepare CAR-T cells capable of recognizing and attacking cancer cells, and administering these cells to a recipient by infusion or the like.

"Chimeric antigen receptor (CAR)" means a fusion protein containing an extracellular domain capable of binding to an antigen such as a cancer antigen, a transmembrane domain, and an intracellular domain having signaling activity. More specifically, it is an artificial antigen receptor in which a single-chain antibody (scFv), formed by linking the variable regions of the light and heavy chains (V_{L} and V_{H}) derived from a monoclonal antibody that recognizes a cell surface antigen, is combined with a transmembrane domain and a signaling domain of a costimulatory molecule involved in T-cell activation. CARs can be roughly classified into first- to third-generation CARs based on the structure and type of the signaling domains contained therein. A first-generation CAR has a signaling domain constituted by only CD3ζ (also known as CD247). A second-generation CAR contains, in the signaling domain, one costimulatory molecule such as CD28 or CD137 (4-1BB) in addition to CD3ζ. A third-generation CAR contains, in the signaling domain, a plurality of costimulatory molecules such as CD28 and CD137 (4-1BB) in addition to CD3ζ. Moreover, a CAR obtained by additionally introducing a genetic modification into any of the above-described CARs to enhance cellular functions may be referred to as a fourth-generation CAR. In T cells expressing a CAR, the CAR recognizes tumor cells and concurrently activates the T cells, and thus, can efficiently kill the target cells. T cells into which a CAR gene has been introduced are referred to as "chimeric antigen receptor T cells (CAR-T cells)".

"CD19" is a signaling molecule involved in the regulation of B-cell (lymphocyte) development, activation, and differentiation, and is known to be expressed on cancer cells such as those of leukemia and lymphoma.

A "signal peptide" refers to an extracellular translocation signal required for secreting a protein biosynthesized through gene expression to the outside of the cell, and is also referred to as a leader peptide or the like. A signal peptide may include a region constituted by hydrophobic amino acids. A signal peptide may be cleaved and removed by a signal peptidase after translation and before translocation to the outside of the cell. A signal peptide sequence may be present at the N-terminus of many secretory proteins and membrane proteins, and may be, for example, 15 to 30 amino acids long. The species from which a signal peptide used is derived may be any species. The species may be human or non-human, for example, an insect cell or a virus, but is preferably human. The type of protein from which the signal peptide is derived is not particularly limited, and examples may include CD8α (SEQ ID NO: 12), immunoglobulin, Oncostatin M, and CD3ε.

A "linker" refers to a peptide that can be inserted between respective portions of a fusion protein, such as the CAR of the present invention, so that the respective portions fused can perform intended functions thereof. The length of the linker is not particularly limited, and examples thereof usually include 3 to 100 amino acids long, and preferably 5 to 50 amino acids long. Typically, as linkers, peptides that contain a large number of amino acids with relatively small side chains, such as serine and glycine, are often used. Examples of the "linker" in a CAR may include a linker between the variable regions of an scFv and a linker between the scFv and the hinge.

Examples of a T cell may include a killer T cell (cytotoxic T cell), a helper T cell, and a regulatory T cell. The T cell may be either a CD8-positive T cell or a CD4-positive T cell. Moreover, T cells can also be classified into a naive T cell, a memory T cell, and an effector T cell, and any of these may be used. It is known that a naive T cell and a memory T cell are abundantly contained in peripheral blood, whereas an effector T cell is scarcely contained in peripheral blood.

As used herein, the term "a plurality of" refers to an integer of 2 or more, for example, an integer from 2 to 10, 2 to 7, 2 to 5, 2 to 4, or 2 to 3. As used herein, the term "several" refers, for example, to from 2 to 10, 2 to 7, 2 to 5, 2 to 4, 2 to 3, or 2.

As used herein, the term "identity (sequence identity)" with respect to an amino acid sequence refers to the percentage (%) of identical amino acid residues relative to the total number of amino acid residues when amino acid sequences of two polypeptides to be compared are aligned, with gaps being appropriately inserted into one or both sequences as necessary so as to maximize the number of identical residues. "Identity (sequence identity)" with respect to a nucleic acid sequence can be determined in the same manner.

As used herein, the term "substitution of an amino acid" refers to a substitution among the 20 amino acids that constitute natural proteins. Preferably, the substitution of an amino acid may be a conservative substitution within a group of amino acids having similar properties, such as charge, side-chain characteristics, polarity, or aromaticity. Examples may include substitutions within a group of uncharged polar amino acids having low-polarity side chains (Gly, Asn, Gln, Ser, Thr, Cys, Tyr), a group of branched-chain amino acids (Leu, Val, Ile), a group of neutral amino acids (Gly, Ile, Val, Leu, Ala, Met, Pro), a group of neutral amino acids having hydrophilic side chains (Asn, Gln, Thr, Ser, Tyr, Cys), a group of acidic amino acids (Asp, Glu), a group of basic amino acids (Arg, Lys, His), and a group of aromatic amino acids (Phe, Tyr, Trp).

A chimeric antigen receptor (CAR) may contain an extracellular region, a hinge, a transmembrane region, and an intracellular region. In one embodiment of the CAR of the present invention, the extracellular region may be linked to the transmembrane region via the hinge.

According to the present invention, the "extracellular region" includes a single chain variable fragment (scFv) capable of binding to CD19. As used herein, the term "capable of binding to CD19" refers to being capable of specifically binding to the extracellular domain of CD19. The extracellular domain of CD19 is not particularly limited as long as it is located extracellularly when present on the cell membrane and is positioned such that the scFv in the CAR of the present invention can bind thereto. Whether or not the scFv is capable of binding to CD19 can be evaluated by contacting the scFv to be tested with a CD19 protein, a portion thereof, or a cell expressing CD19, by a method known in the art, and measuring the binding between these.

As used herein, the term "specifically bind" is not particularly limited, and may mean having a binding affinity between an antigen and an antibody with a KD value of 10⁻⁸ M or less, preferably 10⁻⁹ M or less, and more preferably 10⁻¹⁰ M or less. As a method for measuring the binding affinity, for example, surface plasmon resonance as well as bio-layer interferometry may be employed, and, for example, bio-layer interferometry may suitably be used.

The scFv can be constituted by a heavy chain variable region and a light chain variable region linked by a flexible linker. While a specific linker used is not particularly limited, a GS linker or the like may be used. Moreover, the positions of the heavy chain variable region and the light chain variable region are not particularly limited, and either may be positioned on the N-terminal side or the C-terminal side.

Any scFv can be used, as long as it can bind to CD19, as the scFv capable of binding to CD19 contained in the extracellular region of the CAR of the present invention. In one embodiment, the scFv capable of binding to CD19 may contain the amino acid sequence shown in SEQ ID NO: 11, or an amino acid sequence having at least 90% identity to the amino acid sequence shown in SEQ ID NO: 11. In another embodiment, the scFv usable in the CAR of the present invention may contain (i) a heavy chain variable region and a light chain variable region consisting of an amino acid sequence having at least 90% or more, 95% or more, 98% or more, or 99% or more identity to the amino acid sequence shown in SEQ ID NO: 11, excluding the CDR sequences, or (ii) a heavy chain variable region and a light chain variable region consisting of an amino acid sequence in which one or more amino acids are added, deleted, and/or substituted in the amino acid sequence shown in SEQ ID NO: 11, excluding the CDR sequences.

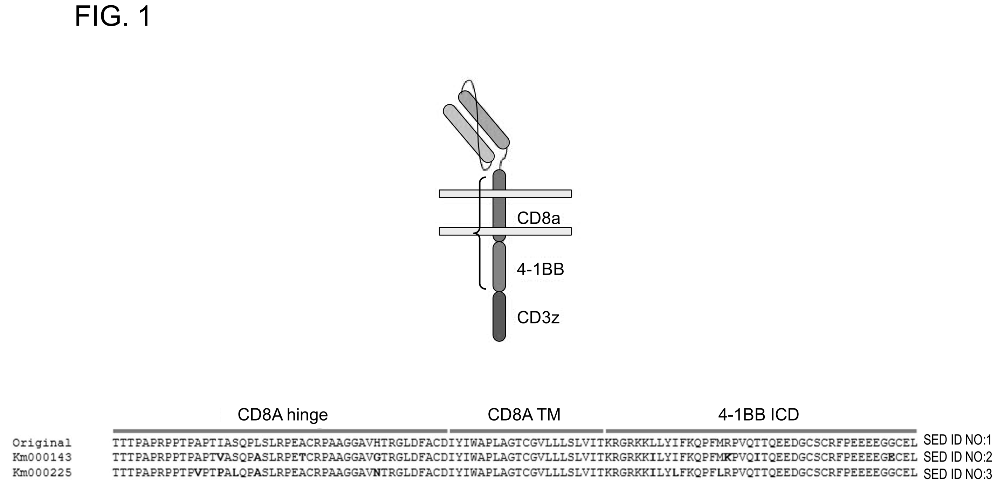

The CAR of the present invention may be a CAR variant that comprises, based on a CAR containing an scFv capable of binding to CD19, a CD8α-derived hinge, a transmembrane domain, a 4-1BB-derived intracellular domain, and a CD3ζ-derived intracellular domain, a CD8α-derived hinge having specific mutations and a 4-1BB-derived intracellular domain (costimulatory domain) having specific mutations. A schematic diagram of an exemplary CAR variant is illustrated in the upper part of FIG. 1. An alignment of the amino acid sequences in a region including a hinge (CD8A hinge), a transmembrane region (CD8A TM), and a costimulatory domain (4-1BB ICD) in the original CAR (Original) and the exemplary CAR variants (km000143 and km000225) is illustrated in the lower part of FIG. 1. The sequence homology among the aligned three amino acid sequences is 92.5%.

As illustrated in the lower part of FIG. 1, the CAR variant km000143 has four residue mutations in the hinge and four residue mutations in the costimulatory domain compared to the original CAR. Moreover, the CAR variant km000225 has five residue mutations in the hinge and three residue mutations in the costimulatory domain compared to the original CAR.

Accordingly, the CAR of the present invention comprises the CD8α-derived hinge having specific mutations. In one embodiment, the hinge contains or consists of the amino acid sequence shown in SEQ ID NO: 5 or 6.

According to the present invention, the "transmembrane region" is a region for stably incorporating the CAR of the present invention into a lipid membrane, and is usually primarily constituted by hydrophobic amino acid residues. As the transmembrane region, a transmembrane domain contained in any membrane protein, or an artificial transmembrane domain mainly containing hydrophobic amino acid residues can be used. Examples of the transmembrane region may include, but are not limited to, CD8, CD28, CD3ε, CD45, CD4, CD5, CD9, CD16, CD22, CD33, CD37, CD64, CD80, CD86, CD134, CD137, CD154, KIRDS2, OX40, CD2, CD27, LFA-1, ICOS, 4-1BB, GITR, CD40L, BAFFR, HVEM, SLAMF7, NKp80, CD160, CD19, IL2Rβ, IL2Ry, IL7Rα, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1, SLAMF4, CD84, CD96, CEACAM1, CRTAM, Ly9, CD160, PSGL1, CD100, SLAMF6, SLAM, BLAME, SELPLG, LTBR, PAG/Cbp, and a transmembrane domain derived from the α chain or β chain of a T cell receptor. The transmembrane region may preferably be a transmembrane domain of CD8α. A specific amino acid sequence of the transmembrane domain of CD8α is not particularly limited, and examples may include an amino acid sequence consisting of or containing the amino acid sequence shown in SEQ ID NO: 10; an amino acid sequence consisting of or containing an amino acid sequence having 90% or more, 95% or more, 98% or more, or 99% or more identity to the amino acid sequence shown in SEQ ID NO: 10; and an amino acid sequence consisting of or containing an amino acid sequence in which one or more amino acids are, deleted, substituted or added in the amino acid sequence shown in SEQ ID NO: 10.

In some embodiments, the CAR may have two or more transmembrane domains, and in this case, these transmembrane domains may be a repetition of the same transmembrane domain, or may be different transmembrane domains from each other.

According to the present invention, the "intracellular region" is a region that transmits an activation signal into the inside of a CAR-expressing cell after the extracellular region of the CAR binds to an antigen molecule on a target cell, and may include a costimulatory domain, a signaling domain, and the like.

As used herein, the term "costimulatory domain" means a domain derived from a costimulatory molecule for enhancing the effector function, cytotoxic activity, cytokine production, proliferation, and/or survival based on the intracellular region including the CD3ζ signaling domain.

As described above, the CAR of the present invention may contain the 4-1BB-derived costimulatory domain having specific mutations. In one embodiment, the costimulatory domain may contain or consist of the amino acid sequence shown in SEQ ID NO: 8 or 9.

In one embodiment, in the CAR of the present invention, the hinge may comprise the amino acid sequence shown in SEQ ID NO: 5, and the costimulatory domain may comprise the amino acid sequence shown in SEQ ID NO: 8. In a more specific embodiment, in the CAR of the present invention, the hinge, the transmembrane region, and the intracellular region (i) comprise or consist of the amino acid sequence shown in SEQ ID NO: 2, (ii) comprise or consist of an amino acid sequence having 90% or more, 95% or more, 98% or more, or 99% or more identity to the amino acid sequence shown in SEQ ID NO: 2, or (iii) comprise or consist of an amino acid sequence in which one or more amino acids are added, deleted, and/or substituted in the amino acid sequence shown in SEQ ID NO: 2 (preferably excluding the hinge and the intracellular region).

In another embodiment, in the CAR of the present invention, the hinge may comprise the amino acid sequence shown in SEQ ID NO: 6, and the costimulatory domain may comprise the amino acid sequence shown in SEQ ID NO: 9. In a more specific embodiment, in the CAR of the present invention, the hinge, the transmembrane region, and the intracellular region (i) comprise or consist of the amino acid sequence shown in SEQ ID NO: 3, (ii) comprise or consist of an amino acid sequence having 90% or more, 95% or more, 98% or more, or 99% or more identity to the amino acid sequence shown in SEQ ID NO: 3, or (iii) comprise or consist of an amino acid sequence in which one or more amino acids are added, deleted, and/or substituted in the amino acid sequence shown in SEQ ID NO: 3 (preferably excluding the hinge and the intracellular region).

In one embodiment, the intracellular region of the CAR of the present invention may further contain a CD3ζ signaling domain. In this case, the intracellular region of the CAR of the present invention may additionally contain only the CD3ζ signaling domain, or may further contain one or more costimulatory domains in addition to the CD3ζ signaling domain.

As used herein, the term "CD3ζ signaling domain" means a domain containing an activation motif, ITAM, which allows the CD3ζ subunit within a T-cell receptor/CD3 complex to trigger various phosphorylation signals upon binding of MHC and an antigen peptide to a T-cell receptor. A specific amino acid sequence of the CD3ζ signaling domain is not particularly limited, and examples may include an amino acid sequence consisting of or containing the amino acid sequence shown in SEQ ID NO: 13; an amino acid sequence consisting of or containing an amino acid sequence having 90% or more, 95% or more, 98% or more, or 99% or more identity to the amino acid sequence shown in SEQ ID NO: 13; and an amino acid sequence consisting of or containing an amino acid sequence in which one or more amino acids are deleted, substituted or added in the amino acid sequence shown in SEQ ID NO: 13. Another example may include a domain in which one or more ITAMs are deleted from any of the CD3ζ signaling domains containing activation motifs, ITAMs. In the intracellular region of the CAR of the present invention, the number of CD3ζ signaling domains is not limited, and may be one or more. For example, the function of the CAR can be enhanced by linking two CD3ζ signaling domains in tandem.

The CAR of the present invention may comprise, in addition to the costimulatory domain and the signaling domain described above, one or more costimulatory domains, and/or one or more signaling domains. Proteins from which the additional signaling domains are derived are not particularly limited, and examples may include signaling domains of CD3ζ, CD28, 4-1BB/CD137, OX-40, CD27, CD40L, GITR, ICOS, CD30, PD-1 (CD279), LFA-1, LIGHT, CD2, CD7, NKG2C (CD94), NK cell activating receptor, BAFFR, BLAME (SLAMF8), BTLA, CD100 (SEMA4D), CD103, CD160 (BY55), CD18, CD19, CD19a, CD247, CD29, CD3δ, CD3ε, CD3γ, CD4, CD40, CD49a, CD49D, CD49f, CD69, CD84, CD8, CD8α, CD8β, CD96 (Tactile), CD11a, CD11b, CD11c, CD11d, CDS, CEACAM1, CRTAM, cytokine receptor, DAP-10, DNAM1(CD226), Fcy receptor, GADS, HVEM (LIGHTR), IA4, ICAM-1, Igα (CD79a), IL-2Rβ, IL-2Rγ, IL-7Rα, integrin, ITGA4, ITGA6, ITGAD, ITGAE, ITGAL, ITGAM, ITGAX, ITGB2, ITGB7, ITGB1, KIRDS2, LAT, CD83-specific ligand, LTBR, Ly9 (CD229), Ly108, MHC class I molecule, NKG2D, NKp30, NKp44, NKp46, NKp80 (KLRF1), PAG/Cbp, PSGL1, SELPLG (CD162), SLAM, SLAMF1, SLAMF4 (CD244), SLAMF6 (NTB-A), SLAMF7, SLP-76, TNF receptor, TNFR2, TNFSF14, Toll ligand receptor, TRANCE/RANKL, VLA1, VLA6 and/or B7-H3 (CD276). As the additional costimulatory domains, any of the signaling regions illustrated above with respect to the signaling domain may be used.

In the intracellular region of the CAR of the present invention, the positions of the signaling domains and the costimulatory domains may not be particularly limited. For example, the intracellular region may contain one or more costimulatory domains on the N-terminal side, and signaling domains on the C-terminal side.

The CAR of the present invention may comprise an additional peptide if necessary. For example, the CAR may comprise an additional linker between the transmembrane region and the intracellular region, or in another position, and may also be a fusion protein in which a marker protein is fused to the C-terminal region or the like. The type of marker protein is not particularly limited, and examples may include fluorescent proteins (such as GFP).

In some embodiments, the CAR of the present invention may comprise a signal peptide on the N-terminal side. For example, the CAR of the present invention may comprise, in this order from the N-terminal side, a signal peptide, an extracellular region, a hinge, a transmembrane region, and an intracellular region.

Examples of the configuration of the CAR of the present invention may include a signal peptide, an scFv capable of binding to CD19, a linker (such as a myc tag), a CD8α-derived hinge having mutations, a transmembrane region, a 4-1BB-derived costimulatory domain having mutations, and a CD3ζ signaling domain. In one embodiment, the CAR of the present invention (i) comprises or consists of the amino acid sequence shown in SEQ ID NO: 16, (ii) comprises or consists of an amino acid sequence having 90% or more, 95% or more, 98% or more, or 99% or more identity to the amino acid sequence shown in SEQ ID NO: 16, or (iii) comprises or consists of an amino acid sequence in which one or more amino acids are added, deleted, and/or substituted in the amino acid sequence shown in SEQ ID NO: 16 (preferably excluding the hinge and the costimulatory domain). In another embodiment, the CAR of the present invention (i) comprises or consists of the amino acid sequence shown in SEQ ID NO: 17, (ii) comprises or consists of an amino acid sequence having 90% or more, 95% or more, 98% or more, or 99% or more identity to the amino acid sequence shown in SEQ ID NO: 17, or (iii) comprises or consists of an amino acid sequence in which one or more amino acids are added, deleted, and/or substituted in the amino acid sequence shown in SEQ ID NO: 17 (preferably excluding the hinge and the costimulatory domain).

CAR-T cells transduced with the CAR of the present invention can induce antigen-specific cytotoxic activity against CD19-expressing cells. CD19 targeted by the CAR of the present invention is known to be expressed in various cells such as cancer cells, and therefore may be effective for the treatment or prevention of diseases involving such cells, including the reduction or elimination of these cells, or the inhibition of the activity thereof.

### 2. Nucleic Acid Sequence Encoding CAR, and CAR Expression Vector

A second aspect of the present invention is a nucleic acid sequence encoding a CAR, and a vector comprising the same (herein also referred to as the "CAR vector"). Specifically, the present invention provides a nucleic acid sequence encoding the CAR of the present invention. The present invention also provides a vector comprising the nucleic acid sequence of the present invention.

The "nucleic acid sequence encoding a CAR" may be any nucleic acid sequence encoding any one of the CARs described in the first aspect, without be limited to the nucleic acid sequence itself in particular. Examples may include a codon-optimized nucleotide sequence, and a nucleotide sequence in which a start codon (ATG) is added at the 5' end.

The CAR vector of the present invention comprises the nucleic acid sequence encoding the CAR. The CAR vector may comprise, in addition to the nucleic acid sequence as a constituent element, constituent elements such as a promoter, a marker gene (selection marker), an enhancer, a terminator, an origin of replication, and a polyA signal, as necessary. When the CAR vector of the present invention is a gene expression vector that additionally contains a promoter and the like, and is capable of expressing the CAR within a cell, this CAR vector is particularly referred to as a CAR expression vector.

As used herein, the term "gene expression vector" refers to a vector that contains a gene or a gene fragment (hereinafter referred to as a "gene or the like") in an expressible state, and encompasses an expression unit capable of controlling the expression of the gene or the like. The gene expression vector may be a plasmid vector or a viral vector.

As used herein, the term "expressible state" refers to a state in which a gene or the like to be expressed is positioned in a region downstream of a promoter and under the control of the promoter. While plasmid vectors, viral vectors, and the like are known as vectors, any of these vectors can be utilized. Usually, a plasmid vector that allows for easy genetic recombination procedures, or a viral vector that enables easy gene introduction into T cells may be used.

The plasmid vector may be, for example, a commercially available expression vector for mammalian cells such as pCI vector or pSI vector of Promega, or a shuttle vector capable of replicating between mammalian cells and bacteria such as *E. coli.*

As the viral vector, various viral vectors can be used, such as retroviral vectors (including an oncoretroviral vector, a lentiviral vector, and a pseudotyped vector), adenoviral vectors, adeno-associated virus (AAV) vectors, simian virus vectors, vaccinia virus vectors, Sendai virus vectors, Epstein-Barr virus (EBV) vectors, and HSV vectors. A replication-deficient viral vector may be used so as not to undergo self-replication within the infected cells.

When a retroviral vector is used, retroviral particles can be produced by selecting a suitable packaging cell and a packaging signal sequence based on the LTR sequences. Examples of the packaging cell may include PG13 (ATCC (R) CRL-10686 (TM)), PA317 (ATCC (R) CRL-9078 (TM)), GP+E-86 and GP+envAm-12 (U.S. Patent No. 5,278,056), and Psi-Crip (1988; doi: 10.1073/pnas.95.21.12516). Retroviral particles can also be produced using 293 cells or 293T cells having high transfection efficiency. Many types of viral vectors, produced based on packaging cells usable for packaging retroviruses and retroviral vectors, are commercially available from a large number of companies.

As used herein, the term "promoter" refers to a gene expression regulatory region capable of controlling the expression of a gene or the like positioned downstream (at the 3' end) in a cell into which a gene expression vector has been introduced. Promoters can be classified into ubiquitous promoters (systemic promoters) and site-specific promoters, based on the location in which a gene or the like under expression control is to be expressed. A ubiquitous promoter is a promoter that controls the expression of a gene or the like targeted (target gene, or the like) in the entire host individual. A site-specific promoter is a promoter that controls the expression of a target gene or the like only in specific cells or tissues. The promoter contained in the gene expression vector of the present invention may be either a ubiquitous promoter or a site-specific promoter, but is preferably capable of inducing the expression in T cells.

Furthermore, promoters are classified into constitutively active promoters, inducible promoters, and temporally specific active promoters based on the timing of expression. A constitutively active promoter can constantly express a target gene or the like in a cell. An inducible promoter can induce the expression of a target gene or the like in a cell at any desired time. A temporally specific active promoter can induce the expression of a target gene or the like in a cell only during a specific stage of development. Since any of these promoters can lead to excessive expression of the target gene in host cells, these can be understood as overexpression-type promoters. The promoter contained in the gene expression vector of the present invention may be preferably a constitutively active promoter that enables long-term persistence of the therapeutic effect.

In the gene expression vector of the present invention, the promoter may be one capable of inducing the expression of the nucleic acid sequence encoding the CAR in cells such as T cells. Since the target cells into which the gene expression vector of the present invention is introduced may be, in principle, mammalian cells, particularly human-derived cells such as human T cells, any promoter capable of expressing the downstream gene in these cells may be used. Examples may include the SFFV promoter, the CMV promoter (CMV-IE promoter), the SV40 early promoter, the RSV promoter, the EF1α promoter, the Ub promoter, the β-actin promoter, and the 5' LTR promoter. For a retroviral vector, the nucleic acid sequence encoding the CAR can be positioned downstream of the 5' LTR promoter to induce the gene expression.

As used herein, the term "marker gene" refers to a gene encoding a marker protein, also designated as a selection marker or a reporter protein. The term "marker protein" refers to a peptide capable of determining, based on the activity thereof, whether or not the marker gene is expressed. The detection of the activity may be performed by directly detecting the activity of the marker protein itself, or by indirectly detecting it via a metabolite generated by the activity of the marker protein, such as a pigment. The detection may be any of biological detection (including detection by binding of a peptide or a nucleic acid such as an antibody or an aptamer), chemical detection (including enzymatic detection), physical detection (including behavioral analytic detection), or sensory detection by a detector (including detection by vision, touch, olfaction, hearing, and taste).

The type of marker protein encoded by the marker gene is not particularly limited, as long as the activity can be detected by a method known in the art. A marker protein with low invasiveness to a transformant may be preferred in the detection. Examples may include a tag peptide, a drug resistance protein, a pigment protein, a fluorescent protein, and a luminescent protein.

As used herein, the term "enhancer" is not particularly limited as long as it can enhance the expression efficiency of a gene or a fragment thereof in the vector.

As used herein, a "terminator" is a sequence capable of terminating the transcription of a gene or the like expressed by the activity of the promoter. The type of terminator is not particularly limited. A terminator derived from the same species as the promoter may be preferred. In a single gene expression control system, a terminator that is paired with the promoter on the genome may particularly be preferred.

### 3. T Cells

A third aspect of the present invention is a T cell. Specifically, the present invention provides a T cell that expresses the CAR of the present invention, or comprises the nucleic acid sequence of the present invention. The T cell is herein referred to also as CAR-T cell.

The T cell is not particularly limited as long as it is that conventionally used for CAR-T cell therapy, and naive T cells and memory T cells may be preferred. The T cell may be, in addition to cells derived from a living body, immortalized cell lines, or cells differentiated from ES cells or induced pluripotent stem cells (iPS cells).

The T cell of the present invention may comprise, in addition to the CAR, a cytokine, a chemokine, a cytokine receptor, a chemokine receptor, a transcription factor, an epigenetic factor, and/or an immune checkpoint molecule, and may also comprise nucleic acids and/or vectors encoding these. Examples of the cytokine or the chemokine may include IL2, IL7, IL12, IL15, IL18, IL21, IL23, GM-CSF, CCL19, and CCL21. The cytokine receptor or the chemokine receptor may be a receptor of any of these secreted factors. These factors may be contained in the CAR expression vector, or may be expressed by an independent expression vector. For example, such a factor may be linked to the CAR via a self-cleaving peptide, such as P2A, within the CAR expression vector. Alternatively, genes encoding a cytokine, a chemokine, a cytokine receptor, a chemokine receptor, a transcription factor, an epigenetic factor, and/or an immune checkpoint molecule can be deleted through genetic modification.

The T cell of the present invention may express a CAR capable of binding to CD19. Therefore, the T cell of the present invention can induce antigen-specific cytotoxic activity against cells expressing CD19. Thus, the T cell of the present invention may be used in CAR-T cell therapy as CAR-T cell.

### 4. Composition and Pharmaceutical Composition

A fourth aspect of the present invention is a composition or a pharmaceutical composition. The present invention provides a composition comprising the CAR of the present invention, and/or the nucleic acid sequence and/or the vector of the present invention. This composition can be used as a pharmaceutical composition for cancer, or for producing the T cell of the present invention. Moreover, the present invention provides a pharmaceutical composition (cell therapy product) comprising the T cell of the present invention. This pharmaceutical composition can be used in the cancer treatment, and can be used in, for example, CAR-T cell therapy.

As used herein, the term "subject" refers to a target to which the composition and the pharmaceutical composition of the present invention are applied, and may be, for example, a tissue, an organ, or an individual. When the subject is an individual, it may be, for example, a mammal, and preferably a human individual. The human individual may be a patient, such as a cancer patient.

As used herein, "subject information" refers to various information regarding the characteristics and conditions of a subject. For example, when the subject is a human individual, the information may include age, body weight, sex, general health status, presence or absence of disease, degree of progression or severity of the disease, drug sensitivity, presence or absence of concurrent medications, and resistance to treatment.

As used herein, the term "treatment" refers to the alleviation or elimination of symptoms associated with a disease, and/or the prevention or suppression of disease progression, and the cure of the disease.

The type of "cancer" is not limited, and examples may include adenocarcinoma, squamous cell carcinoma, small cell cancer, and large cell cancer. Specific examples of the cancer may include malignant melanoma, oral cancer, laryngeal cancer, pharyngeal cancer, thyroid cancer, lung cancer, breast cancer, esophageal cancer, gastric cancer, small intestinal cancer, colorectal cancer (including colon cancer and rectal cancer), liver cancer, pancreatic cancer, biliary tract cancer (including gallbladder cancer and bile duct cancer), renal cancer, bladder cancer, prostate cancer, testicular cancer, endometrial cancer, cervical cancer, uterine corpus cancer, ovarian cancer, skin cancer, brain tumor, head and neck cancer, glioma, neuroblastoma, mesothelioma, osteosarcoma, soft tissue sarcoma, and hematologic cancer (e.g., lymphoma, myeloma). Examples of hematologic cancer may include leukemia (e.g., B-cell leukemia, acute lymphoblastic leukemia, chronic lymphocytic leukemia), lymphoma (e.g., non-Hodgkin lymphoma), and myeloma (e.g., multiple myeloma). Cancers suitable for the application of the pharmaceutical composition (cell therapy product) of the present invention are cancers associated with CD19, such as hematologic cancers. It is known that CAR-T cells are effective even in chemoresistant cancers and recurrent cancers after bone marrow transplantation, and therefore, the pharmaceutical composition of the present invention can be applied to such refractory cancers and recurrent cancers.

The constituent elements of the composition and the pharmaceutical composition of the present invention will now be described. The composition and the pharmaceutical composition of the present invention may comprise, as essential constituent elements, one or more active ingredients, and a solvent and/or a carrier. These constituent elements will now be specifically described.

The composition of the present invention may comprise, as an essential active ingredient, the CAR of the present invention, and/or the nucleic acid sequence and/or the vector of the present invention.

The pharmaceutical composition of the present invention may comprise the T cell of the present invention as an essential active ingredient. The pharmaceutical composition of the present invention may comprise one or more types of T cells.

The amount of the active ingredient contained in the composition and the pharmaceutical composition of the present invention is not particularly limited. In general, the amount may vary depending on the type of active ingredient, the dosage form, and the types of other constituent elements described below, such as a solvent and a carrier. Accordingly, the amount may be appropriately determined in view of the respective conditions. It is sufficient that a single-dose cell therapy product contains an effective amount of the active ingredient. However, when a large amount of the cell therapy product and/or pharmaceutical composition needs to be administered to a subject in order to achieve the pharmacological effect of the active ingredient, the administration may be divided into several doses to reduce the burden on the subject. In this case, it is sufficient that the total amount of the active ingredient administered is the effective amount.

Moreover, for example, the composition of the present invention containing the nucleic acid sequence and/or the vector (such as an expression vector) of the present invention may be used as a composition to be used for producing the T cell of the present invention.

The term "effective amount" refers to an amount necessary for exerting the function as the active ingredient, while causing little or no harmful side effects to a subject. This effective amount may vary depending on various conditions, including information about the subject, the route of administration, the number of administrations, and the like. Accordingly, when the composition and/or the pharmaceutical composition of the present invention is used as a medicament, the amount of the active ingredient may ultimately be determined at the discretion of a physician, a pharmacist, or the like.

The amount of T cell contained in the pharmaceutical composition of the present invention may be, for example, 10⁵ cells to 10¹⁰ cells, or 10⁶ cells to 10⁹ cells, and may be preferably 10⁷ cells to 10⁸ cells.

The composition and the pharmaceutical composition of the present invention may contain an additional active ingredient. In this case, the type of the additional active ingredient is not particularly limited. Examples may include another CAR (e.g., a CAR capable of binding to a tumor-cell surface antigen other than CD19), and CAR-expressing T cells.

The composition and the pharmaceutical composition of the present invention may contain a pharmaceutically acceptable solvent as needed. The term "pharmaceutically acceptable solvent" refers to a solvent usually used in the field of formulation techniques. Examples may include water, an aqueous solution, and an organic solvent. Examples of the aqueous solution may include physiological saline, an isotonic solution containing glucose or another auxiliary agent, phosphate buffer, phosphate buffered saline, sodium acetate buffer, and a solution of glycol or ethanol. Examples of the auxiliary agent may include D-sorbitol, D-mannose, D-mannitol, sodium chloride, and, in addition, a nonionic surfactant having a low concentration, and polyoxyethylene sorbitan fatty acid ester. An example of the organic solvent may include ethanol.

The composition and the pharmaceutical composition of the present invention may contain a pharmaceutically acceptable carrier as needed. The term "pharmaceutically acceptable carrier" refers to an additive usually used in the field of formulation techniques. Examples may include an excipient and human serum albumin.

Examples of the excipient may include sugars such as monosaccharides, disaccharides, cyclodextrins, and polysaccharides, inorganic acid salts such as hydrochloride, hydrobromide, phosphate, and sulfate, salts of organic acids such as acetate, propionate, malonate, and benzoate, metal salts, citric acid, tartaric acid, glycine, polyethylene glycol, kaolin, silicic acid, and combinations thereof.

In addition to the above, the composition and the pharmaceutical composition may also contain, as needed, a solubilizing agent, a suspending agent, a diluent, a dispersing agent, a surfactant, a soothing agent, a stabilizer, an absorption enhancer, an extending agent, a preservative, an antiseptic, an antioxidant, a buffering agent, a tonicity agent, and the like that are usually used in pharmaceutical compositions and the like.

A carrier may be used not only to avoid or suppress degradation of the active ingredient by enzymes or the like in the body of the subject, but also to facilitate formulation and administration and to maintain the dosage form and medicinal effect, and may be used as appropriate when needed.

The dosage form of the pharmaceutical composition (cell therapy product) of the present invention is not particularly limited. The dosage form may be any form as long as it is delivered to a target site within the body of the subject without being inactivated.

The specific dosage form may vary depending on the administration method described below. The administration method can be broadly classified into parenteral and oral administration, and parenteral administration may be preferred.

When the administration method is parenteral, a preferred dosage form may be a liquid preparation that allows direct administration to the target site or systemic administration via the circulatory system. A preferable example of the liquid preparation may be an injection preparation. An injection preparation can be formulated by mixing the active ingredient with a solvent and, as appropriate, an excipient, a suspending agent, a surfactant, a stabilizer, a pH adjuster, and the like, to obtain a unit dosage form required for generally accepted pharmaceutical implementation.

The method of applying the pharmaceutical composition of the present invention is not particularly limited, and the route of administration may not be limited, and for example, parenteral administration may be suitably employed. Parenteral administration can be further subdivided into systemic administration and local administration. Examples of local administration may include intradermal administration, intramuscular administration, subcutaneous administration, intraperitoneal administration, intranasal administration, intratumoral administration, tissue administration, and organ administration, and examples of systemic administration by parenteral administration may include intravascular administration, such as intravenous administration (intravenous injection), intra-arterial administration, and intralymphatic administration. A preferred route of administration may be intravenous administration, which may be performed by infusion (for example, by a single intravenous infusion).

The pharmaceutical composition of the present invention can be used for the treatment and/or prevention of a disease such as cancer (particularly a disease in which CD19 is involved). Accordingly, the present invention provides a method for treating and/or preventing a disease in a subject, comprising administering, to the subject, an effective amount of the pharmaceutical composition of the present invention. In this case, the target disease may be, for example, cancer, and the subject may be a cancer patient. The treatment and/or prevention method may be conducted as CAR-T cell therapy.

The present invention also provides use of the CAR, the nucleic acid sequence and/or the vector encoding the CAR, the T cell, and the composition or the pharmaceutical composition of the present invention in the production of a medicament for treating and/or preventing a disease such as cancer (particularly a disease in which CD19 is involved).

The invention further provides the CAR, the nucleic acid sequence and/or the vector encoding the CAR, the T cell, and the composition or the pharmaceutical composition of the present invention for use in the treatment and/or prevention of a disease such as cancer (particularly a disease in which CD19 is involved).

### 5. Method for Producing T cells

In a fifth aspect, the present invention provides a method for producing T cells. CAR-T cells can be produced using a vector, or by employing a method such as transposon method. The production method of the present invention may comprise a CAR-encoding nucleic acid introduction step.

The method for producing T cell of the present invention may comprise the CAR-encoding nucleic acid introduction step as an essential step, and may include, as optional steps, a cell isolation step and an expansion culture step. The respective steps will now be specifically described.

The "cell isolation step" is an optional step in the method of the present invention, and is a step of isolating T cells to be used as a host. For example, when the cells are peripheral blood mononuclear cells, this step may be a peripheral blood mononuclear cell isolation step in which peripheral blood mononuclear cells are isolated from peripheral blood obtained from the subject. In this case, the purpose of this step is to isolate peripheral blood mononuclear cells containing T cells.

As used herein, the term "peripheral blood mononuclear cells (PBMCs)" refers to cells or cell populations that are isolated from the peripheral blood of a human or an animal, and may include monocytes and/or lymphocytes such as T cells.

The isolation method can be appropriately selected depending on the type of cells to be isolated. Although peripheral blood mononuclear cells are described below as an example, the present invention is not limited thereto, and any of the cells illustrated in the third aspect may be isolated in this step.

When peripheral blood mononuclear cells are used as the cells, the isolation method is not particularly limited. For example, peripheral blood mononuclear cells can be isolated by density-gradient centrifugation or by a hemolysis method. Density-gradient centrifugation can be performed by layering a diluted whole-blood sample over a medium and centrifuging the resultant. As the medium, Ficoll-Hypaque (R) or the like can be used, and an example includes a medium adjusted to a density of approximately 1.077 g/mL. As a result of density-gradient centrifugation, a mononuclear cell fraction can be separated as an intermediate layer from erythrocytes, granulocytes, and plasma. The hemolysis method is a method for removing erythrocytes using a hypotonic solution, but this method does not remove granulocytes. Accordingly, this step can be generally carried out by density-gradient centrifugation.

The "expansion culture step" is an optional step in the method of the present invention, and is a step of expanding the isolated cells by culture to increase the number of cells. This step can be carried out simultaneously with, or subsequent to the cell isolation step.

The method employed in this step can be appropriately selected depending on the type of cells used, and is not particularly limited.

For example, when the cells are peripheral blood mononuclear cells, the step can be carried out by stimulating the cells with an anti-CD3 antibody. The anti-CD3 antibody used in this step is not particularly limited as long as it is capable of stimulating peripheral blood mononuclear cells, and for example, a soluble anti-CD3 antibody or cells expressing a membrane-bound anti-CD3 antibody may be used. Furthermore, the proliferation of peripheral blood mononuclear cells stimulated with the anti-CD3 antibody may be maintained by culturing the cells in the presence of a cytokine such as IL-2.

This step can be carried out to promote the proliferation of specific cells in the isolated cells, and to improve the efficiency of CAR introduction in the subsequent introduction step.

The "introduction step" is an essential step in the method of the present invention, and is a step of introducing, into the isolated cells, a nucleic acid sequence encoding the CAR of the present invention or a vector containing the nucleic acid sequence. This step can be carried out after the cell isolation step, if performed, and simultaneously with or subsequently to the expansion culture step, if performed.

The cells into which a nucleic acid sequence encoding the CAR has been introduced in this step can be used, for example when the cells are peripheral blood mononuclear cells, as CAR-T cells in CAR-T cell therapy.

The method for introducing a nucleic acid sequence encoding the CAR, for example a vector, into peripheral blood mononuclear cells is not particularly limited. The method can be appropriately selected depending on the form of the nucleic acid, the type of vector, the type of cells, and the like. A method for introducing a nucleic acid sequence encoding the CAR using a vector will now be described.

When the vector is a viral vector, methods for infecting cells with the virus are well known in the art. Upon the introduction of a viral vector, a functional substance for enhancing viral infection efficiency, such as fibronectin or fibronectin fragment (for example, RetroNectin (R), that is, a fibronectin fragment having a heparin-binding domain, or Vecofusin-1 (R)), may be used.

An example of a viral infection method using RetroNectin is as follows. After treating a cell-culture plate with RetroNectin, the bottom surface of the plate may be blocked with a 2% BSA/PBS solution for 30 minutes. Then, after washing with PBS, a retrovirus liquid derived from PG13 packaging cells may be loaded onto the plate, and the resultant culture plate may be centrifuged at 32°C and 2,000 g for 2 hours. After the centrifugation, the viral solution may be removed, and the resulting cells may be seeded onto a plate.

When the vector is a non-viral vector such as a plasmid, gene introduction methods (transfection methods) known in the art, such as that described in Green & Sambrook, 2012, Molecular Cloning: A Laboratory Manual Fourth Ed., Cold Spring Harbor Laboratory Press, Cold Spring Harbor, New York, or the like may be employed. Examples may include lipofection, electroporation, microinjection, the calcium phosphate method, the DEAE-dextran method, and particle bombardment.

The cells into which the nucleic acid sequence encoding the CAR has been introduced in this step can usually be directly used, but if necessary, only the cells into which the nucleic acid sequence encoding the CAR has been introduced may be isolated. The isolation method to be employed here is not particularly limited, and separation and/or enrichment may be performed, for example, based on the marker gene contained in the vector.

### Examples

The present invention will now be more specifically described by way of examples, and it is noted that the present invention is not limited by these examples.

### [Example 1] Design of CAR

### (1) Design of CAR Expression Vector

As a CAR expression vector, a lentiviral vector was used.

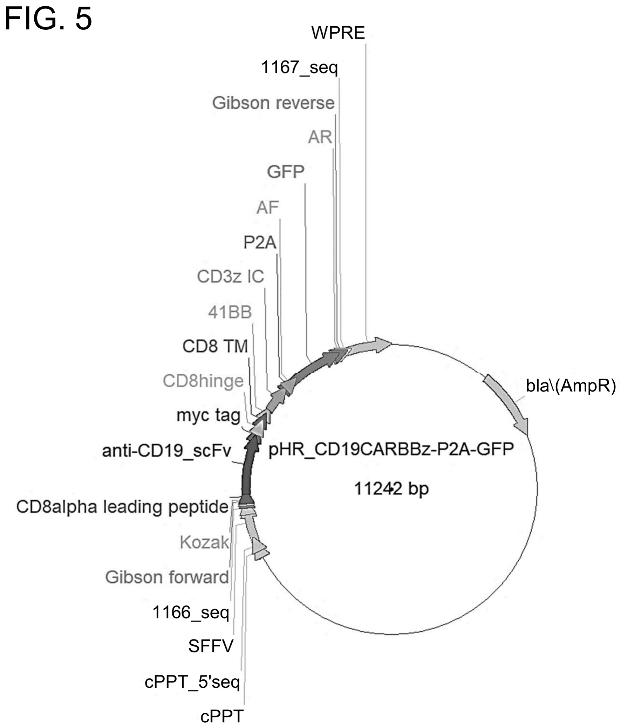

To produce the lentiviral vector, a plasmid vector was first designed by inserting a nucleotide sequence encoding a CAR downstream of the SFFV promoter in pHR-SFFV (Addgene accession number: 79121, as of the filing date). The design details of each encoded CAR are shown in Table 1 below. As an example of the structure of the plasmid vector used as the basis, the structure of the plasmid vector "pHR_CD19CARBBz-P2A-GFP" that encodes a CAR BBz is shown in FIG. 5. Each CAR was designed as a fusion protein that contains a Myc tag (indicated as "myc tag" in FIG. 5) between the scFv (indicated as "anti-CD19_scFv" in FIG. 5) and the hinge and transmembrane region (indicated as "CD8 hinge" and "CD8 TM" in FIG. 5), and has a P2A peptide (SEQ ID No: 14) and GFP (EGFP; SEQ ID No: 15) fused at the C-terminus. Variants km000143 and km000225 contained mutations in the CD8α hinge and the 4-1BB costimulatory domain (indicated as "CD8 hinge" and "41BB" in FIG. 5).

**Table 1: Design of CARs**

| Name | Signal Peptide | scFv | Linker | Hinge | Transmembrane Region | Costimulatory Domain | Signaling Domain | Total Length |
|---|---|---|---|---|---|---|---|---|
| BBz | CD8α (SEQ ID NO:12) | CD19 scFv (SEQ ID NO:11) including GS linker | myc tag | CD8α (SEQ ID NO:4) | CD8α (SEQ ID NO:10) | 4-1BB (SEQ ID NO:7) | CD3ζ (SEQ ID NO:13) | SEQ ID NO:21 |
| km000143 | CD8α (SEQ ID NO:12) | CD19 scFv (SEQ ID NO:11) including GS linker | myc tag | Mutant CD8α (SEQ ID NO:5) | CD8α (SEQ ID NO:10) | Mutant 4-1BB (SEQ ID NO:8) | CD3ζ (SEQ ID NO:13) | SEQ ID NO:16 |
| km000225 | CD8α (SEQ ID NO:12) | CD19 scFv (SEQ ID NO:11) including GS linker | myc tag | Mutant CD8α (SEQ ID NO:6) | CD8α (SEQ ID NO:10) | Mutant 4-1BB (SEQ ID NO:9) | CD3ζ (SEQ ID NO:13) | SEQ ID NO:17 |
| 28z | CD8α (SEQ ID NO:12) | CD19 scFv (SEQ ID NO:11) including GS linker | myc tag | CD28 | CD28 | CD28 | CD3ζ (SEQ ID NO:13) | SEQ ID NO:20 |

Next, each plasmid vector and the packaging mix (pCMVR8.74 (Addgene accession number: 22036), pMD2.G (Addgene accession number: 12259), and pAdVAntage (TM) (Promega Corp.)) were cotransfected for infection into packaging cells (Thermo Fisher Scientific; 293FT cells) to produce a CAR-expressing lentiviral vector capable of inducing CAR expression. The lentiviral vector was prepared in accordance with the protocol provided by the manufacturer of the packaging mix.

### (2) Introduction into Cells and Culture

As the host cells, human primary CD8⁺ T cells (STEMCELL Technologies, ST-200-0164) were used. Each lentiviral vector was used to infect 1 × 10⁶ cells.

48 hours after infection, the cells were cultured for 5 to 7 days in a culture medium (medium obtained by adding, to X-VIVO 15 medium (Lonza, 04-418Q) supplemented with 5% human serum and 100 µM 2-mercaptoethanol, human IL-2 (PeproTech) at a final concentration of 25 U/mL and human IL-7 and human IL-15 (both from Miltenyi Biotec) at final concentrations of 100 ng/mL).

The infected T cells were passaged in the culture medium at a cell density of 5.0 × 10⁴ cells/mL and cultured until 5 days after infection. As a control, T cells that had not been infected with the lentiviral vector and had been similarly cultured in the culture medium for 5 days were used.

### (3) Measurement of CAR Introduction Efficiency

The obtained cells were detected for the presence or absence of GFP expression using a flow cytometer (CytoFLEX S; Beckman Coulter). The flow-cytometric analysis was performed in accordance with the protocol provided by the manufacturer.

The determination of the presence or absence of the GFP signal was made based on a threshold by which all cells of the same type not infected with the lentiviral vector ("WT") were determined to be negative.

For each condition, at least 5,000 cells were subjected to measurement, and the experiment was performed repeatedly three times.

The proportion of GFP-positive cells, i.e., CAR-positive cells, in each population of infected T-cells was examined, and found to be 40 to 60% with no substantial differences. Accordingly, it was confirmed that the designed CAR variants were successfully introduced into the T cells without any issues.

### [Example 2] Cytotoxic Activity of CAR-expressing T cells

In this example, the cytotoxic activity of T cells expressing the CAR variants (km000143 and km000225) designed in Example 1 was assessed using a serial killing assay.

The design of a CAR expression vector, and the preparation of and the infection with a lentiviral vector were conducted in the same manner as in Example 1.

The T cells selected after infection were passaged by suspending these cells in culture medium at a cell density of 50 × 10⁴ cells/mL, and seeding 2 mL of the suspension into each well of a 6-well plate. The cells were then cultured in the culture medium, with passaging performed to keep the density of 1 × 10⁶ cells/mL to 4 × 10⁶ cells/mL, until day 12 after infection. As controls, T cells not infected with the lentiviral vector but similarly cultured in the culture medium for 12 days (WT), T cells expressing the CAR (BBz) from which the variants were derived, and T cells expressing a CAR having a different structure (28z) were used.

Target cells at 5 × 10⁴ cells/well and T cells at 0.625 × 10⁴ cells/well obtained 6 days after infection were seeded into a 96-well plate (E:T ratio = 1:4) and co-cultured for 72 hours in co-culture medium (X-VIVO15 medium supplemented with 5% human serum and 100 µM 2-mercaptoethanol). As the target cells, Nalm-6 cells (RIKEN BRC) transduced with a luciferase gene expression vector (hereinafter referred to as Nalm-Luc) were used.

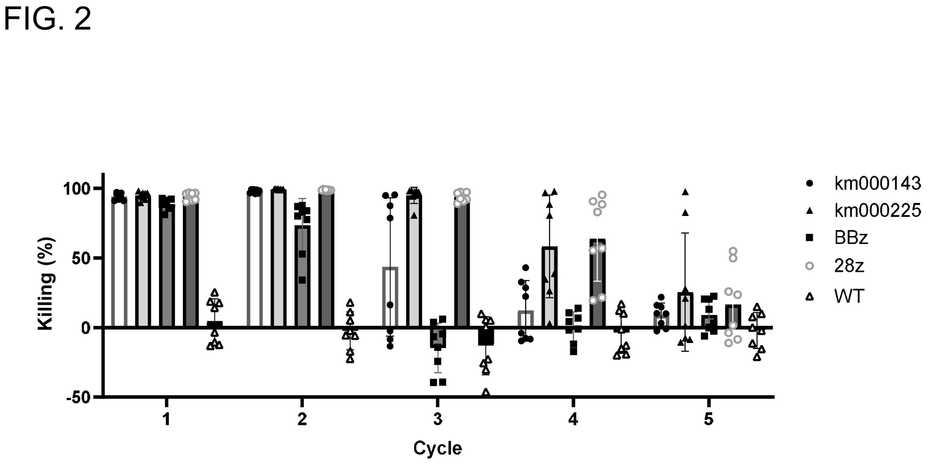

After 72 hours of co-culture, half the volume of the co-cultured sample and the target cells at 5 × 10⁴ cells/well were seeded into a new 96-well plate, and the resultant was co-cultured for an additional 48 or 72 hours, which cycle was repeated four times. The remaining volumes of the co-cultured samples from the respective cycles, and the co-culture sample from the final cycle, were used to measure cytotoxic activity against Nalm-Luc cells (CD19-positive) (respectively illustrated as Cycles 1 to 5 in FIG. 2).

The cytotoxic activity was measured by adding a cell lysate containing luciferin substrate (OneGlo, Promega) to the co-cultured sample, and measuring luciferase activity using a microplate reader (SH-9000Lab). In the measurement of cytotoxic activity, the target cells alone were seeded at 5 × 10⁴ cells/well (without T cells) into a fresh 96-well plate, simultaneously with the start of the co-culture, to be similarly cultured. The measurement result obtained from these target cells was defined as 0% cytotoxic activity (killing), and a luminescence value of 0 was defined as 100% cytotoxic activity (killing). Based on these definitions, the cytotoxic activity (reduction rate of Nalm cells) in each co-culture condition was calculated. The experiment was performed repeatedly three times under each condition.

The results are illustrated in FIG. 2.

FIG. 2 is a graph illustrating cytotoxic activity obtained in the first co-culture (Cycle 1) and cytotoxic activity obtained in the subsequent co-cultures using the serially diluted cultures (Cycles 2 to 5) against Nalm-Luc cells of each type of transduced CAR.

Regarding cytotoxic activity in the first co-culture, all the T cells transduced with the CAR variants exhibited higher cytotoxic activity compared to the T cells without CAR transduction ("WT" in FIG. 2). In the co-cultures of Cycles 2 to 5, no cytotoxic activity was observed in the T cells without CAR transduction ("WT" in FIG. 2). Also in the repeated co-cultures, all the CAR-transduced T cells showed cytotoxic activity, but as the number of cycles increased, the variants (km000143 and km000225) maintained the cytotoxic activity as compared to the BBz from which the variants were derived, and maintained a level of cytotoxic activity comparable to that of 28z. It is known that 28z excels in short-term cytotoxicity, whereas BBz is excellent in activity persistence.

### [Example 3] Persistence of CAR-expressing T Cells

In this example, the *in vivo* persistence of T cells expressing the CAR variants (km000143 and km000225) designed in Example 1 was investigated by detecting the expression of the combinations of cell surface markers CD45RA and CD62L, which serve as indicators of differentiation, by antibody staining and flow cytometry. As cell differentiation progressed, the expression of markers changed in the order of: CD45RA-positive and CD62L-positive (Naive/SCM), CD45RA-negative and CD62L-positive (CM), CD45RA-negative and CD62L-negative (EM), and CD45RA-positive and CD62L-positive (TEMRA). SCM, CM, EM, and TEMRA respectively stand for Stem-Cell Memory T cells, Central Memory T cells, Effector Memory T cells, and Effector Memory T cells expressing CD45RA.

Specifically, 6.25 ×10³ CAR-T cells/well prepared in Example 1 and 1.25 × 10⁴ CD19-expressing K562 cells were co-cultured in a 96-well round-bottom plate for 48 hours, and then stained with CD8-PE, CD62L-BV421, and CD45RA-APC to measure the expression patterns of CD62L and CD45RA in CD8⁺ GFP⁺ cells. The cells were then divided into four subsets, Naive/SCM, CM, EM, and TEMRA, and the results were graphed.

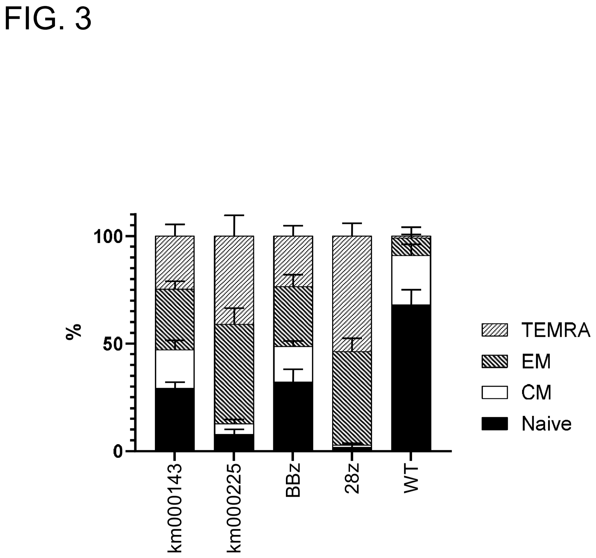

The results are illustrated in FIG. 3. All T cells expressing the CAR variants (km000143 and km000225) showed a similar degree of differentiation to the BBz from which the variants were derived, and compared to 28z, the proportions of Naive SCM and CM were higher, and the degree of differentiation was lower, which suggests that these T cells were superior in persistence.

### [Example 4] In Vivo Validation of Designed CARs

In this example, Nalm-luc cells were intravenously injected into NSG mice, and the CAR T cells were further intravenously administered four days later, and thus, the efficacy of the CAR T cells was evaluated *in vivo* by monitoring whole-body luminescence values and survival rates over time.

As target cells, 1 × 10⁶ Nalm-Luc cells were transplanted into the tail vein of immunodeficient NSG mice (The Jackson Laboratory Japan, Inc.; strain name NOD.Cg-Prkdcscid Il2rgtm1Wjl/SzJ).

4 days after the transplantation of target cells, 2 × 10⁶ CD19 CAR-expressing T cells (variant km000143, km000225, or BBz) or PBS as a control were intravenously injected into these mice (day 4). On days 7, 11, 14, 18, 21, 25, 28, 32, and 35, luciferin substrate was intraperitoneally administered, and whole-body luminescence value was measured using IVIS. Additionally, the survival rate up to day 35 was determined.

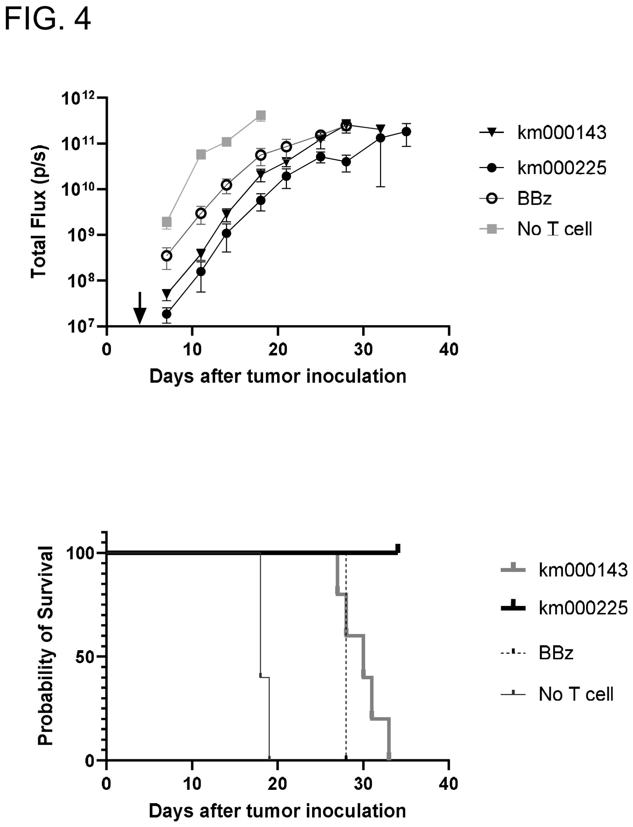

The results are illustrated in FIG. 4. In FIG. 4, the upper graph illustrates the whole-body luminescence value, and the lower graph illustrates the survival rate. As a result, regarding the luminescence values, which indicate the abundance of cancer cells, the variant km000225 showed lower values than BBz on day 18. Furthermore, the survival period was extended in both the variants km000143 and km000225 compared to BBz. In this manner, it was demonstrated that the CAR-T cells expressing the variants km000143 and km000225 possess cytotoxic activity equivalent to or higher than that of the BBz from which the variants were derived.

### Sequence Information:

SEQ ID NO: 1 (original (CD8A hinge + CD8A TM + 4-1BB ICD)) 
TTTPAPRPPTPAPTIASQPLSLRPEACRPAAGGAVHTRGLDFACDIYIWAPLAGTCGVL
LLSLVITKRGRKKLLYIFKQPFMRPVQTTQEEDGCSCRFPEEEEGGCEL
SEQ **ID** NO: 2 (km000143 (CD8A hinge + CD8A TM + 4-1BB ICD)) 
TTTPAPRPPTPAPTVASQPASLRPETCRPAAGGAVGTRGLDFACDIYIWAPLAGTCGV
LLLSLVITKRGRKKILYIFKQPFMKPVQITQEEDGCSCRFPEEEEGECEL
SEQ **ID** NO: 3 (km000225 (CD8A hinge + CD8A TM + 4-1BB ICD)) 
TTTPAPRPPTPVPTPALQPASLRPEACRPAAGGAVNTRGLDFACDIYIWAPLAGTCGV
LLLSLVITKRGRKKILYLFKQPFLRPVQTTQEEDGCSCRFPEEEEGGCEL
SEQ ID NO: 4 (CD8 hinge)
   TTTPAPRPPTPAPTIASQPLSLRPEACRPAAGGAVHTRGLDFACD
SEQ ID NO: 5 (mutant CD8 hinge (km000143))
   TTTPAPRPPTPAPTVASQPASLRPETCRPAAGGAVGTRGLDFACD
SEQ ID NO: 6 (mutant CD8 hinge (km000225))
   TTTPAPRPPTPVPTPALQPASLRPEACRPAAGGAVNTRGLDFACD
SEQ ID NO: 7 (4-1BB signaling domain)
   KRGRKKLLYIFKQPFMRPVQTTQEEDGCSCRFPEEEEGGCEL
SEQ ID NO: 8 (mutant 4-1BB signaling domain (km000143))
   KRGRKKILYIFKQPFMKPVQITQEEDGCSCRFPEEEEGECEL
SEQ ID NO: 9 (mutant 4-1BB signaling domain (km000225))
   KRGRKKILYLFKQPFLRPVQTTQEEDGCSCRFPEEEEGGCEL
SEQ ID NO: 10 (CD8 transmembrane)
   IYIWAPLAGTCGVLLLSLVIT
SEQ ID NO: 11 (CD19 scFv (FMC63)) 
DIQMTQTTSSLSASLGDRVTISCRASQDISKYLNWYQQKPDGTVKLLIYHTSRLHSGV
PSRFSGSGSGTDYSLTISNLEQEDIATYFCQQGNTLPYTFGGGTKLEITGGGGSGGGGS
GGGGSEVKLQESGPGLVAPSQSLSVTCTVSGVSLPDYGVSWIRQPPRKGLEWLGVIW
GSETTYYNSALKSRLTIIKDNSKSQVFLKMNSLQTDDTAIYYCAKHYYYGGSYAMDY
WGQGTSVTVSS
SEQ ID NO: 12 (CD8 leading peptides)
   MALPVTALLLPLALLLHAARP
SEQ ID NO: 13 (CD3z ICD) 
RVKFSRSADAPAYKQGQNQLYNELNLGRREEYDVLDKRRGRDPEMGGKPRRKNPQ
EGLYNELQKDKMAEAYSEIGMKGERRRGKGHDGLYQGLSTATKDTYDALHMQALP
PR
SEQ ID NO: 14 (P2A peptide)
   GSGATNFSLLKQAGDVEENPGP
SEQ ID NO: 15 (EGFP) 
MVSKGEELFTGVVPILVELDGDVNGHKFSVSGEGEGDATYGKLTLKFICTTGKLPVP
WPTLVTTLTYGVQCFSRYPDHMKQHDFFKSAMPEGYVQERTIFFKDDGNYKTRAEV
KFEGDTLVNRIELKGIDFKEDGNILGHKLEYNYNSHNVYIMADKQKNGIKVNFKIRHN
IEDGSVQLADHYQQNTPIGDGPVLLPDNHYLSTQSALSKDPNEKRDHMVLLEFVTAA
GITLGMDELYK
SEQ ID NO: 16 (km000143, CAR) 
MALPVTALLLPLALLLHAARPDIQMTQTTSSLSASLGDRVTISCRASQDISKYLNWYQ
QKPDGTVKLLIYHTSRLHSGVPSRFSGSGSGTDYSLTISNLEQEDIATYFCQQGNTLPY
TFGGGTKLEITGGGGSGGGGSGGGGSEVKLQESGPGLVAPSQSLSVTCTVSGVSLPDY
GVSWIRQPPRKGLEWLGVIWGSETTYYNSALKSRLTIIKDNSKSQVFLKMNSLQTDDT
AIYYCAKHYYYGGSYAMDYWGQGTSVTVSSEQKLISEEDLNGAATTTPAPRPPTPAP
TVASQPASLRPETCRPAAGGAVGTRGLDFACDIYIWAPLAGTCGVLLLSLVITKRGRK
KILYIFKQPFMKPVQITQEEDGCSCRFPEEEEGECELRVKFSRSADAPAYKQGQNQLY
NELNLGRREEYDVLDKRRGRDPEMGGKPRRKNPQEGLYNELQKDKMAEAYSEIGM
KGERRRGKGHDGLYQGLSTATKDTYDALHMQALPPR
SEQ ID NO: 17 (km000225, CAR) 
MALPVTALLLPLALLLHAARPDIQMTQTTSSLSASLGDRVTISCRASQDISKYLNWYQ
QKPDGTVKLLIYHTSRLHSGVPSRFSGSGSGTDYSLTISNLEQEDIATYFCQQGNTLPY
TFGGGTKLEITGGGGSGGGGSGGGGSEVKLQESGPGLVAPSQSLSVTCTVSGVSLPDY
GVSWIRQPPRKGLEWLGVIWGSETTYYNSALKSRLTIIKDNSKSQVFLKMNSLQTDDT
AIYYCAKHYYYGGSYAMDYWGQGTSVTVSSEQKLISEEDLNGAATTTPAPRPPTPVP
TPALQPASLRPEACRPAAGGAVNTRGLDFACDIYIWAPLAGTCGVLLLSLVITKRGRK
KILYLFKQPFLRPVQTTQEEDGCSCRFPEEEEGGCELRVKFSRSADAPAYKQGQNQLY
NELNLGRREEYDVLDKRRGRDPEMGGKPRRKNPQEGLYNELQKDKMAEAYSEIGM
KGERRRGKGHDGLYQGLSTATKDTYDALHMQALPPR
SEQ ID NO: 18 (km000143, CAR, nucleotides) 
ATGGCCTTACCAGTGACCGCCTTGCTCCTGCCGCTGGCCTTGCTGCTCCACGCCGC
CAGGCCGGACATCCAGATGACACAGACTACATCCTCCCTGTCTGCCTCTCTGGGA
GACAGAGTCACCATCAGTTGCAGGGCAAGTCAGGACATTAGTAAATATTTAAATT
GGTATCAGCAGAAACCAGATGGAACTGTTAAACTCCTGATCTACCATACATCAAG
ATTACACTCAGGAGTCCCATCAAGGTTCAGTGGCAGTGGGTCTGGAACAGATTAT
TCTCTCACCATTAGCAACCTGGAGCAAGAAGATATTGCCACTTACTTTTGCCAACA
GGGTAATACGCTTCCGTACACGTTCGGAGGCGGCACCAAGCTGGAGATCACAGGT
GGCGGTGGCTCGGGCGGTGGTGGGTCGGGTGGCGGCGGATCTGAGGTGAAACTG
 
CAGGAGTCAGGACCTGGCCTGGTGGCGCCCTCACAGAGCCTGTCCGTCACATGCA
CTGTCTCAGGGGTCTCATTACCCGACTATGGTGTAAGCTGGATTCGCCAGCCTCCA
CGAAAGGGTCTGGAGTGGCTGGGAGTAATATGGGGTAGTGAAACCACATACTAT
AATTCAGCTCTCAAATCCAGACTGACCATCATCAAGGACAACTCCAAGAGCCAAG
TTTTCTTAAAAATGAACAGTCTGCAAACTGATGACACAGCCATTTACTACTGTGCC
AAACATTATTACTACGGTGGTAGCTATGCTATGGACTACTGGGGCCAAGGAACCT
CAGTCACCGTCTCCTCAGAACAAAAACTCATCTCAGAAGAAGATCTGAATGGGGC
CGCAACTACAACGCCGGCTCCAAGACCGCCGACGCCGGCACCAACCGTGGCGAG
CCAACCCGCTTCCCTTCGACCAGAGACTTGTCGACCTGCTGCTGGGGGAGCGGTG
GGTACACGAGGACTCGATTTCGCTTGTGACATATACATCTGGGCTCCTCTGGCTGG
CACTTGCGGAGTGCTTCTGCTGAGTCTGGTTATTACCAAACGGGGCAGAAAGAAG
ATCCTGTATATCTTCAAACAACCATTTATGAAGCCAGTACAAATTACTCAAGAGG
AAGATGGGTGTAGCTGCCGATTTCCAGAAGAAGAAGAAGGAGAGTGTGAACTGA
GAGTGAAGTTCAGCAGGAGCGCAGACGCCCCCGCGTACAAGCAGGGCCAGAACC
AGCTCTATAACGAGCTCAATCTAGGACGAAGAGAGGAGTACGATGTTTTGGACAA
GAGACGTGGCCGGGACCCTGAGATGGGGGGAAAGCCGAGAAGGAAGAACCCTCA
GGAAGGCCTGTACAATGAACTGCAGAAAGATAAGATGGCGGAGGCCTACAGTGA
GATTGGGATGAAAGGCGAGCGCCGGAGGGGCAAGGGGCACGATGGCCTTTACCA
GGGTCTCAGTACAGCCACCAAGGACACCTACGACGCCCTTCACATGCAGGCCCTG
CCCCCTCGC
SEQ ID NO: 19 (km000225, CAR, nucleotides) 
ATGGCCTTACCAGTGACCGCCTTGCTCCTGCCGCTGGCCTTGCTGCTCCACGCCGC
CAGGCCGGACATCCAGATGACACAGACTACATCCTCCCTGTCTGCCTCTCTGGGA
GACAGAGTCACCATCAGTTGCAGGGCAAGTCAGGACATTAGTAAATATTTAAATT
GGTATCAGCAGAAACCAGATGGAACTGTTAAACTCCTGATCTACCATACATCAAG
ATTACACTCAGGAGTCCCATCAAGGTTCAGTGGCAGTGGGTCTGGAACAGATTAT
TCTCTCACCATTAGCAACCTGGAGCAAGAAGATATTGCCACTTACTTTTGCCAACA
GGGTAATACGCTTCCGTACACGTTCGGAGGCGGCACCAAGCTGGAGATCACAGGT
GGCGGTGGCTCGGGCGGTGGTGGGTCGGGTGGCGGCGGATCTGAGGTGAAACTG
 
CAGGAGTCAGGACCTGGCCTGGTGGCGCCCTCACAGAGCCTGTCCGTCACATGCA
CTGTCTCAGGGGTCTCATTACCCGACTATGGTGTAAGCTGGATTCGCCAGCCTCCA
CGAAAGGGTCTGGAGTGGCTGGGAGTAATATGGGGTAGTGAAACCACATACTAT
AATTCAGCTCTCAAATCCAGACTGACCATCATCAAGGACAACTCCAAGAGCCAAG
TTTTCTTAAAAATGAACAGTCTGCAAACTGATGACACAGCCATTTACTACTGTGCC
AAACATTATTACTACGGTGGTAGCTATGCTATGGACTACTGGGGCCAAGGAACCT
CAGTCACCGTCTCCTCAGAACAAAAACTCATCTCAGAAGAAGATCTGAATGGGGC
CGCAACCACTACTCCAGCTCCTAGACCGCCTACCCCGGTCCCTACTCCGGCACTGC
AGCCTGCTTCTCTTAGACCTGAGGCATGCAGGCCCGCCGCTGGCGGGGCAGTCAA
TACCAGAGGACTCGACTTCGCTTGCGATATATACATTTGGGCACCTCTGGCAGGT
ACGTGCGGAGTGCTCTTGCTGAGTTTGGTGATTACTAAACGCGGCAGGAAAAAA
TACTGTACCTGTTCAAACAACCATTCTTGCGGCCAGTACAGACAACGCAAGAGGA
GGATGGGTGTAGCTGCCGATTCCCAGAGGAGGAAGAAGGAGGCTGTGAGTTGAG
AGTGAAGTTCAGCAGGAGCGCAGACGCCCCCGCGTACAAGCAGGGCCAGAACCA
GCTCTATAACGAGCTCAATCTAGGACGAAGAGAGGAGTACGATGTTTTGGACAAG
AGACGTGGCCGGGACCCTGAGATGGGGGGAAAGCCGAGAAGGAAGAACCCTCAG
GAAGGCCTGTACAATGAACTGCAGAAAGATAAGATGGCGGAGGCCTACAGTGAG
ATTGGGATGAAAGGCGAGCGCCGGAGGGGCAAGGGGCACGATGGCCTTTACCAG
GGTCTCAGTACAGCCACCAAGGACACCTACGACGCCCTTCACATGCAGGCCCTGC
CCCCTCGC
SEQ ID NO: 20 (CD19 CAR (28z)) 
MALPVTALLLPLALLLHAARPDIQMTQTTSSLSASLGDRVTISCRASQDISKYLNWYQ
QKPDGTVKLLIYHTSRLHSGVPSRFSGSGSGTDYSLTISNLEQEDIATYFCQQGNTLPY
TFGGGTKLEITGGGGSGGGGSGGGGSEVKLQESGPGLVAPSQSLSVTCTVSGVSLPDY
GVSWIRQPPRKGLEWLGVIWGSETTYYNSALKSRLTIIKDNSKSQVFLKMNSLQTDDT
AIYYCAKHYYYGGSYAMDYWGQGTSVTVSSEQKLISEEDLNGAAIEVMYPPPYLDN
EKSNGTIIHVKGKHLCPSPLFPGPSKPFWVLVVVGGVLACYSLLVTVAFIIFWVRSKRS
RLLHSDYMNMTPRRPGPTRKHYQPYAPPRDFAAYRSRVKFSRSADAPAYKQGQNQL
 
YNELNLGRREEYDVLDKRRGRDPEMGGKPRRKNPQEGLYNELQKDKMAEAYSEIG
MKGERRRGKGHDGLYQGLSTATKDTYDALHMQALPPR
SEQ ID NO: 21 (CD19 CAR (BBz, original)) 
MALPVTALLLPLALLLHAARPDIQMTQTTSSLSASLGDRVTISCRASQDISKYLNWYQ
QKPDGTVKLLIYHTSRLHSGVPSRFSGSGSGTDYSLTISNLEQEDIATYFCQQGNTLPY
TFGGGTKLEITGGGGSGGGGSGGGGSEVKLQESGPGLVAPSQSLSVTCTVSGVSLPDY
GVSWIRQPPRKGLEWLGVIWGSETTYYNSALKSRLTIIKDNSKSQVFLKMNSLQTDDT
AIYYCAKHYYYGGSYAMDYWGQGTSVTVSSEQKLISEEDLNGAATTTPAPRPPTPAP
TIASQPLSLRPEACRPAAGGAVHTRGLDFACDIYIWAPLAGTCGVLLLSLVITKRGRK
KLLYIFKQPFMRPVQTTQEEDGCSCRFPEEEEGGCELRVKFSRSADAPAYKQGQNQL
YNELNLGRREEYDVLDKRRGRDPEMGGKPRRKNPQEGLYNELQKDKMAEAYSEIG
MKGERRRGKGHDGLYQGLSTATKDTYDALHMQALPPR
SEQ ID NO: 22 (CD19 CAR (28z), nucleotides) 
ATGGCCTTACCAGTGACCGCCTTGCTCCTGCCGCTGGCCTTGCTGCTCCACGCCGC
CAGGCCGGACATCCAGATGACACAGACTACATCCTCCCTGTCTGCCTCTCTGGGA
GACAGAGTCACCATCAGTTGCAGGGCAAGTCAGGACATTAGTAAATATTTAAATT
GGTATCAGCAGAAACCAGATGGAACTGTTAAACTCCTGATCTACCATACATCAAG
ATTACACTCAGGAGTCCCATCAAGGTTCAGTGGCAGTGGGTCTGGAACAGATTAT
TCTCTCACCATTAGCAACCTGGAGCAAGAAGATATTGCCACTTACTTTTGCCAACA
GGGTAATACGCTTCCGTACACGTTCGGAGGCGGCACCAAGCTGGAGATCACAGGT
GGCGGTGGCTCGGGCGGTGGTGGGTCGGGTGGCGGCGGATCTGAGGTGAAACTG
CAGGAGTCAGGACCTGGCCTGGTGGCGCCCTCACAGAGCCTGTCCGTCACATGCA
CTGTCTCAGGGGTCTCATTACCCGACTATGGTGTAAGCTGGATTCGCCAGCCTCCA
CGAAAGGGTCTGGAGTGGCTGGGAGTAATATGGGGTAGTGAAACCACATACTAT
AATTCAGCTCTCAAATCCAGACTGACCATCATCAAGGACAACTCCAAGAGCCAAG
TTTTCTTAAAAATGAACAGTCTGCAAACTGATGACACAGCCATTTACTACTGTGCC
AAACATTATTACTACGGTGGTAGCTATGCTATGGACTACTGGGGCCAAGGAACCT
CAGTCACCGTCTCCTCAGAACAAAAACTCATCTCAGAAGAAGATCTGAATGGGGC
 
CGCAATTGAGGTTATGTACCCACCTCCCTACCTCGATAACGAGAAATCTAATGGC
ACCATCATCCACGTAAAAGGGAAGCATCTGTGTCCTAGTCCTCTGTTCCCTGGTCC
TTCAAAACCCTTTTGGGTGCTGGTGGTTGGTGGAGTCCTGGCTTGCTATAGCT
TGCTAGTAACAGTGGCCTTTATTATTTTCTGGGTGAGGAGTAAGAGGAGCAGGCT
CCTGCACAGTGACTACATGAACATGACTCCCCGCCGCCCCGGGCCCACCCGCAAG
CATTACCAGCCCTATGCCCCACCACGCGACTTCGCAGCCTATCGCTCCAGAGTGA
AGTTCAGCAGGAGCGCAGACGCCCCCGCGTACAAGCAGGGCCAGAACCAGCTCT
ATAACGAGCTCAATCTAGGACGAAGAGAGGAGTACGATGTTTTGGACAAGAGAC
GTGGCCGGGACCCTGAGATGGGGGGAAAGCCGAGAAGGAAGAACCCTCAGGAA
GGCCTGTACAATGAACTGCAGAAAGATAAGATGGCGGAGGCCTACAGTGAGATT
GGGATGAAAGGCGAGCGCCGGAGGGGCAAGGGGCACGATGGCCTTTACCAGGGT
CTCAGTACAGCCACCAAGGACACCTACGACGCCCTTCACATGCAGGCCCTGCCCC
CTCGC
SEQ ID NO: 23 (CD19 CAR (BBz, original), nucleotides)

ATGGCCTTACCAGTGACCGCCTTGCTCCTGCCGCTGGCCTTGCTGCTCCACGCCGC
CAGGCCGGACATCCAGATGACACAGACTACATCCTCCCTGTCTGCCTCTCTGGGA
GACAGAGTCACCATCAGTTGCAGGGCAAGTCAGGACATTAGTAAATATTTAAATT
GGTATCAGCAGAAACCAGATGGAACTGTTAAACTCCTGATCTACCATACATCAAG
ATTACACTCAGGAGTCCCATCAAGGTTCAGTGGCAGTGGGTCTGGAACAGATTAT
TCTCTCACCATTAGCAACCTGGAGCAAGAAGATATTGCCACTTACTTTTGCCAACA
GGGTAATACGCTTCCGTACACGTTCGGAGGCGGCACCAAGCTGGAGATCACAGGT
GGCGGTGGCTCGGGCGGTGGTGGGTCGGGTGGCGGCGGATCTGAGGTGAAACTG
CAGGAGTCAGGACCTGGCCTGGTGGCGCCCTCACAGAGCCTGTCCGTCACATGCA
CTGTCTCAGGGGTCTCATTACCCGACTATGGTGTAAGCTGGATTCGCCAGCCTCCA
CGAAAGGGTCTGGAGTGGCTGGGAGTAATATGGGGTAGTGAAACCACATACTAT
AATTCAGCTCTCAAATCCAGACTGACCATCATCAAGGACAACTCCAAGAGCCAAG
TTTTCTTAAAAATGAACAGTCTGCAAACTGATGACACAGCCATTTACTACTGTGCC
AAACATTATTACTACGGTGGTAGCTATGCTATGGACTACTGGGGCCAAGGAACCT
CAGTCACCGTCTCCTCAGAACAAAAACTCATCTCAGAAGAAGATCTGAATGGGGC

CGCAACTACAACGCCGGCTCCAAGACCGCCGACGCCGGCACCAACCATAGCGTCT
CAACCCCTTTCCCTTCGACCAGAGGCTTGTCGACCTGCTGCTGGGGGAGCGGTGC
ACACACGAGGACTCGATTTCGCTTGTGACATATACATCTGGGCTCCTCTGGCTGGC
ACTTGCGGAGTGCTTCTGCTGAGTCTGGTTATTACCAAACGGGGCAGAAAGAAAC
TCCTGTATATATTCAAACAACCATTTATGAGACCAGTACAAACTACTCAAGAGGA
AGATGGCTGTAGCTGCCGATTTCCAGAAGAAGAAGAAGGAGGATGTGAACTGAG
AGTGAAGTTCAGCAGGAGCGCAGACGCCCCCGCGTACAAGCAGGGCCAGAACCA
GCTCTATAACGAGCTCAATCTAGGACGAAGAGAGGAGTACGATGTTTTGGACAAG
AGACGTGGCCGGGACCCTGAGATGGGGGGAAAGCCGAGAAGGAAGAACCCTCAG
GAAGGCCTGTACAATGAACTGCAGAAAGATAAGATGGCGGAGGCCTACAGTGAG
ATTGGGATGAAAGGCGAGCGCCGGAGGGGCAAGGGGCACGATGGCCTTTACCAG
GGTCTCAGTACAGCCACCAAGGACACCTACGACGCCCTTCACATGCAGGCCCTGC
CCCCTCGC

All publications, patents, and patent applications cited herein are incorporated by reference in their entirety.

## Claims

1. A chimeric antigen receptor (CAR) comprising: a single chain variable fragment (scFv) capable of binding to CD19, a hinge, a transmembrane region, and an intracellular region,
wherein the hinge comprises the amino acid sequence shown in SEQ ID NO: 5 or 6, and
the intracellular region comprises a costimulatory domain comprising the amino acid sequence shown in SEQ ID NO: 8 or 9.

2. The CAR according to claim 1, wherein the hinge comprises the amino acid sequence shown in SEQ ID NO: 5, and the costimulatory domain comprises the amino acid sequence shown in SEQ ID NO: 8.

3. The CAR according to claim 2, wherein the hinge, the transmembrane region, and the intracellular region comprises the amino acid sequence shown in SEQ ID NO: 2, or an amino acid sequence having at least 90% identity to the amino acid sequence shown in SEQ ID NO: 2.

4. The CAR according to claim 1, wherein the hinge comprises the amino acid sequence shown in SEQ ID NO: 6, and the costimulatory domain comprises the amino acid sequence shown in SEQ ID NO: 9.

5. The CAR according to claim 4, wherein the hinge, the transmembrane region, and the intracellular region comprises the amino acid sequence shown in SEQ ID No: 3, or an amino acid sequence having at least 90% identity to the amino acid sequence shown in SEQ ID NO: 3.

6. The CAR according to claim 1, wherein the scFv capable of binding to CD19 comprises the amino acid sequence shown in SEQ ID NO: 11, or an amino acid sequence having at least 90% identity to the amino acid sequence shown in SEQ ID NO: 11.

7. The CAR according to claim 1, wherein the intracellular region further comprises a CD3ζ signaling domain.

8. The CAR according to claim 1, comprising the amino acid sequence shown in SEQ ID NO: 16, or an amino acid sequence having at least 90% identity to the amino acid sequence shown in SEQ ID NO: 16.

9. The CAR according to claim 1, comprising the amino acid sequence shown in SEQ ID NO: 17, or an amino acid sequence having at least 90% identity to the amino acid sequence shown in SEQ ID NO: 17.

10. A nucleic acid sequence encoding the CAR according to claim 1.

11. A vector comprising the nucleic acid sequence according to claim 10.

12. A T cell that expresses the CAR according to claim 1, or comprises the nucleic acid sequence according to claim 10.

13. A composition comprising the CAR according to claim 1, the nucleic acid sequence according to claim 10, or the vector according to claim 11.

14. A pharmaceutical composition comprising the T cell according to claim 12.

15. The pharmaceutical composition according to claim 14, for use in the treatment of cancer.
